(19) Europäisches Patentamt
European Patent Office
Office européen des brevets

(11) Publication number : **0 382 734 B1**

# EUROPEAN PATENT SPECIFICATION

(12)

(45) Date of publication of patent specification :
**30.12.92 Bulletin 92/53**

(51) Int. Cl.$^5$ : **G01N 33/533,** G01N 33/53,
G01N 33/543, G01N 21/64

(21) Application number : **88906854.0**

(22) Date of filing : **03.08.88**

(86) International application number :
**PCT/GB88/00642**

(87) International publication number :
**WO 89/01155 09.02.89 Gazette 89/04**

(54) PHOTOGRAPHIC IMMUNOFLUORESCENT ASSAY.

(30) Priority : **04.08.87 GB 8718374**
**22.04.88 GB 8809610**

(43) Date of publication of application :
**22.08.90 Bulletin 90/34**

(45) Publication of the grant of the patent :
**30.12.92 Bulletin 92/53**

(84) Designated Contracting States :
**AT BE CH DE FR GB IT LI LU NL SE**

(56) References cited :
**EP-A- 0 165 072**
**WO-A-86/07456**
**US-A- 3 973 129**
**J.Clin.Pathol.(1980) 33: 171 - 176**
**Journal of Immunological Methods, 103 (1987), p. 9-14 (H. LABROUSSE et al.): "A method for the quantification of a colored orfluorescent signal in enzyme immunoassays by photodensitometry";**
**Clinical Chemistry, Vol. 30, No. 5, 1984, p. 806-807, (G.H.G. THORPE et al.): "Photographic Monitoring of EnhancedLuminescent Immunoassays";**
**Clinical Chemistry, Vol. 31, No. 8, 1985, p. 1335-1341 (G.H.G. THORPE et al.): "Phenols as Enhancers of the ChemiluminescentHorseradish Peroxidase-Luminol-Hydrogen Peroxide Reaction: Application in Luminescence-Monitored EnzymeImmunoassays";**
**Analytical Biochemistry, Vol. 144, (1985), p. 537-541 (B.W. FALK et al.): "Fluorescent Monitoring of Proteins during SodiumDodecyl Sulfate-Polyacrylamide Gel Electrophoresis and Estern Blotting";**

(56) References cited :
**Society for Experimental Biology and Medicine, Vol. 113, 1963 (F. PARONETTO): "The Fluorescent Antibody Technique Applied toTitration and Identification of Antigens in Solutions or Antisera", pages 394-397;**
**Serum Protein Abnorm. Diagr. Clin. Asp. (Editors S.E. Ritzmann, J.C.Daniels) 1975: (R.M. NAKAMURA et al.) "FluorescentAntibody Assays", pages 135-147;**

(73) Proprietor : **Darougar, Sohrab**
**2 Digby Place**
**Croydon, CR0 5QR (GB)**

(72) Inventor : **Darougar, Sohrab**
**2 Digby Place**
**Croydon, CR0 5QR (GB)**

(74) Representative : **Cresswell, Thomas Anthony et al**
**J.A. Kemp & Co. 14 South Square Gray's Inn London WC1R 5EU (GB)**

Jouve, 18, rue Saint-Denis, 75001 PARIS

## Description

This invention is concerned with improvements in or relating to fluorescent assays.

In particular, the present invention relates to a process for conducting fluorescent assays for materials in biological samples and to a means for use in such assays.

Immunoassays and other assay methods which take advantage of the specific binding reaction between a specific binding reagent such as an antibody or antigen and its specific binding partner such as an antigen or antibody are generally known as are such methods in which the presence or absence in a sample of the specific binding agent is detected by use of a fluorescent label. In essence, such assay methods comprise contacting a known specific binding partner with a sample suspected to contain a specific binding agent, permitting the specific binding partner and agent to interact and detecting the interaction by use of a fluorescent labelling technique.

One example of such a process is a direct immunoassay for the detection of antigens in tissues or cells according to which an immobilised sample containing the suspected antigen is contacted with a corresponding antibody which has been conjugated with a fluorochrome, the fluorescently-labelled antibody being immobilised on a support; areas on the support of enhanced fluorescence indicate where the antigen-antibody reaction has taken place and thus the presence, in the sample, of the suspected antigen.

As well as this direct assay method, there is also available an indirect (sandwich) immunoassay method in which a serum or other body fluid which may contain, for example, an antibody to be detected, is first contacted with the complementary antigen immobilised on a support followed by washing to remove unreacted material; any of the antibody present reacts with the immobilised antigen binding the antibody to it. The immobilised antigen/antibody complex is then contacted with appropriate anti-serum containing antibodies conjugated with the fluorochrome; areas of enhanced fluorescence on the support indicate the presence of the suspected antibody.

A similar indirect method is available for the detection of antigens, again in body fluids, but more usually in tissue or cells, employing the corresponding immobilised antibody.

Other direct and indirect methods for the detection of antigens and antibodies or other specific binding agents using their corresponding specific binding partners are available and the principle is well known to those skilled in the art.

A particular micro-immunofluorescent sandwich technique has previously been reported Forsey and Darougar, Journal of Clinical Pathology, 33 pp 171-176 (1980). in which antigens are immobilised in the form of micro-dots on a microscope slide. The test is conduced by contacting the microscope slide with the test sample, for instance a body fluid or, tissue or cell suspension. The use of a sophisticated photomicroscope for the study of fluorescent samples is known but this involves microphotography at a magnification of say 400 x with a correspondingly reduced optical field of view, and it is not practicable for rapid assessment of the results of e.g. diagnostic test. Similar techniques are referred to, for example, in the following papers:

Wang and Grayston, American Journal of Ophthalmology, pp 367-374, September 1970.

Dryer et al., British Journal of Venereal Diseases 48 pp 452-459. (1972).

Terharne et al., Journal of Clinical Pathology, 30 pp 510-517 (1977).

Darougar et al., British Journal of Ophthamology, 62 pp 503-508 (1978).

Terharne et al., British Journal of Venereal Diseases 54 pp 403-408 (1978).

Forsey and Darougar, British Journal of Ophthalomology 68 pp 409-411 (1984).

Forsey et al., Journal of Infection 12 pp 145-152 (1986).

This micro-immunofluorescent technique can be used for example for the detection of antibodies against any of chlamydia, adenoviruses, herpes simplex virus, human immunodeficiency virus (AIDS), hepatitis B, syphilis and mycoplasma.

The results of the above mentioned assay methods are commonly assessed by technical staff viewing a slide through a microscope, a tedious and subjective procedure requiring a high level of training and experience.

Labrousse et al. [J. Immunol Methods, 103, 9-14 (1987)] describe a solution-phase immunofluorescent assay in which the results are seconded photographically but assessed by photodensitometry.

It is an object of the present invention to provide an improved substrate binding fluorescent assay method.

Surprisingly I have found that objectively assessable results can be obtained rapidly by a simple photographic technique.

The invention provides a fluorescent assay method according to claim 1 below.

It has now surprisingly been found that both the accuracy and the ease of reading the results of such assay methods can be enhanced by conducting the assay as set out in the claims.

The invention also provides a fluorescent assay method comprising the steps of subjecting a sample selectively stained with a fluorochrome to radiation of exciting wavelengths so that the fluorochrome is excited to emit radiation at its emitting wavelengths, and recording images of the excited sample on a photographic film by photography as defined herein.

The process of the present invention obviates the need to use a microscope to collect sufficient light for the human eye to distinguish the results of the control and test spots and has the additional benefits of speed especially in handling multiple tests simultaneously, and of providing a permanent record.

In accordance with the present invention, the test will be conducted on a substrate, such as a microscope slide, containing at least one and usually a number of test patches. Each test patch consists of an area for receiving a sample fluid and a plurality of spots. Each spot is a "micro-dots" either of a control substance or a test substance. The spots containing a test substance (hereafter "test spots") will comprise a specific binding partner immobilised on the substrate, where necessary in association with additional agents essential for detection of the reaction between the specific binding partner and the specific binding agent to be detected. Usually the test spots will comprise an antigen or antibody and an agent for binding the antigen or antibody to the substrate.

The test patch also comprises at least one control spot; this is present to provide a negative control and it is expected that the control spot will not react or will only react slightly with any component of the sample under test. If a reaction of the control spot is observed to be similar to that of a test spot, or if the control spot becomes bright this suggests that there is a defect in the test patch, the sample tested or in the test methodology so that the result is null. The control spot will usually consist of all the components used in the formation of a test spot except for the specific binding partner intended to detect a specific binding agent in the test sample.

The exact nature of both the control and test spots will depend upon the nature of the specific binding agent to be detected and the nature of the available specific binding partners complementary thereto as well as the means available for fluorescently labelling any test spot which has reacted with, or to which is adhering, a specific binding agent in the test sample. Such considerations are generally well known to those skilled in the art who will be able to identify suitable specific binding partners for detecting particular specific binding agents in a sample, means for immobilising the specific binding partners on a suitable subject and means for fluorescently labelling the test spots after reaction with the specific binding reagents. The skilled person will also be aware of the conditions necessary to stimulate fluorescence in the particular fluorescent labels selected for use in the test process.

The photographic recording step is critical to the present invention and there are various photographic methods which will be available to those skilled in the art for conducting this step. In the invention, the fluorescence is recorded phtotographically at a magnification of 1 to 10 x to provide a negative or positive image which is directly readable by the unaided human eye. It is particularly preferred to use instant development film to provide a positive image which may be read by the unaided human eye; more preferably the image is produced at from 1 to 5 x and most preferably at 1.5 to 5 x magnification.

Materials for use in the present invention, particularly microscope slides containing at least one test patch, for instance, 2, 4, 6, 8 or 10 test patches, each containing a control spot and at least one for instance 2, 3, 5, 7 or 9 test spots may be produced as illustrated in the references mentioned above.

The maximum number of test spots in a patch is limited by the minimum detectable fluorescence required to provide a photographic record, by the size of the support member bearing the test patches and by the number of test patches on the support member. On a microscope slide containing an array of 6x3 test patches it has been possible to use up to 18 test spots per patch and this may be further increased with improved spotting techniques.

The invention also provides fluorescent assay method comprising the steps of subjecting a sample selectively stained with a fluorochrome to radiation of exciting wavelengths so that the fluorochrome is excited to emit radiation at its emitting wavelengths, and photographically recording images of the excited sample as hereinbefore described.

The exciting radiation is for example in the range of ultraviolet (UV), Uv-violet, blue-violet, blue and green. The corresponding exciting wavelengths are for example:

UV      ca.365 nm.
UV-violet      390-420 nm.
Blue-violet      395-440 nm.
Blue      450-490 nm.
Green      515-565 nm.

The sample may be derived from humans or animals.

Examples of fluorescent assays include for example assays for antigens, antibodies, DNA, nucleic acids, biogenic amines e.g. catecholamine, primary amines, thrombocytes, lysines, histones, lymphocytes, leucocytes, cell nuclei, cell walls, mucus, albumen, cellulose, bone tissue, polychromes, osteons, chromosomes as well as chlamydia, viruses and bacteria.

In embodiments where the fluorescent assay in an indirect immunoassay for the detection of antigen-antibody reactions, the provision of the selectively stained sample comprises the steps of contacting a serum or other body fluid which may contain an antibody or antigen to be detected, with a complementary immobilised antigen or antibody, respectively; and then contacting the immobilised material with an appropriate anti-serum conjugated with the fluorochrome.

The complementary antigen or antibody may be immobilised in the form of discrete micro-dots on a slide The diameter of the micro-dots is for example 0.5-3mm.

Each plurality of the discrete micro-dots may contain a respective different complementary antibody or antigen, to provide a simultaneous assay for plurality of different antigen or antibodies.

Preferably the photographic film used is a fast monochromatic film up to for example 20,000 ASA. The exposure is for example from 1 to 60 seconds e.g. from 2 to 20 seconds, and the aperture of from f2.8 to f22, e.g. from f16 to f22. One preferred exposure is 2 seconds at f5.6 with 20,000 ASA film.

The invention also comprehends apparatus constructed, arranged and adapted to operate in carrying out a method according to the invention.

There now follows a description to be read with reference to the accompanying drawings of apparatus embodying the invention. This description which is also illustrative of method aspects of the invention is given by way of example only and not by way of limitation thereof.

In the accompanying drawings:

Figure 1 shows an outside front view of the apparatus embodying the invention;

Figure 2 shows a side view corresponding to Figure 1;

Figure 3 is a diagrammatic view illustrating the optical arrangement of the apparatus;

Figures 4 and 5 illustrate a locking and shutter mechanism of the apparatus;

Figure 6 shows a plan view of a slide supporting assembly of the apparatus; and

Figure 7 shows a drawing of a photograph of a slide.

The apparatus comprises a casing 10 comprising a rear enclosure 12 housing a transformer (not shown) an adjustable timer 14, a power supply switch 16 and a push button switch 18, all of which are mounted on a front panel 20 of the rear enclosure 12.

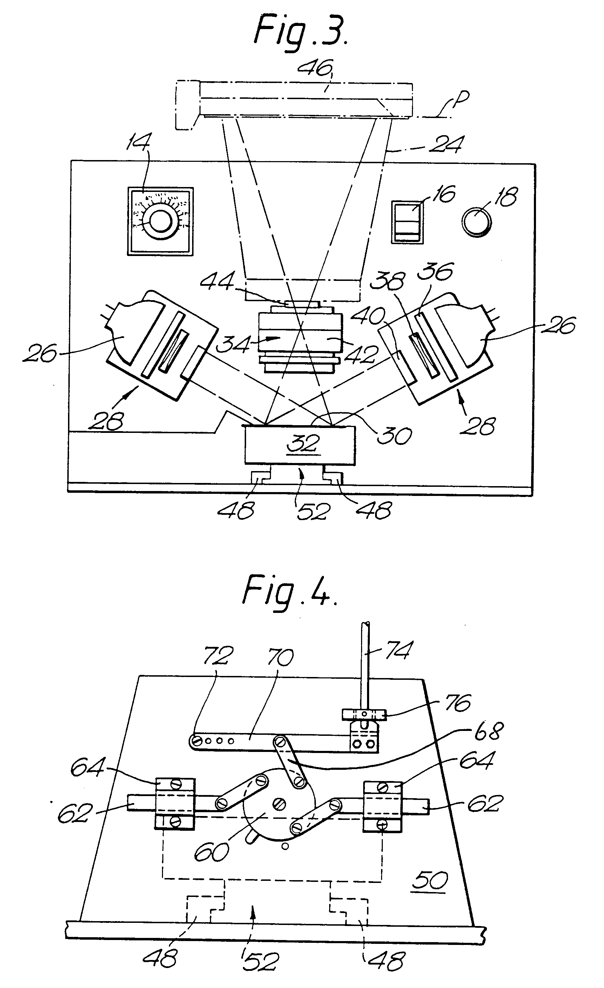

The housing also comprises a light-tight front enclosure 22 on which is mounted a camera 24. The enclosure 22 also houses two symmetrically arranged projector lamps 26 (Figure 3) each followed by an incident lens assembly 28. In the operation of the apparatus a transparent slide 30 with a fluorochrome stained sample thereon is supported in the enclosure 22 directly below the camera 24, by removable support assembly 32; each incident lens assembly 28 is provided between its lamp 26 and the slide 30. An objective lens assembly 34 is mounted immediately below the camera 24 between the camera 24 and the slide 30.

In the operation of the apparatus a parallel beam of ligth from each lamp 26 impinges on the slide 30 <u>via</u> the incident lens assembly 28 and are reflected into the camera 24 <u>via</u> the objective lens assembly 34.

Each lamp 26 is a 100 watt tungsten halogen projector lamp type A1/231 (ANSI code ETP) with a dichroic backing; the lamp is adapted to generate radiation at the fluorochrome's exciting wavelengths. Each lens assembly 28 comprises a heat filter 36, followed by a bi-concave lens 38 and an exciter filter 40, adapted to selectively transmit the exciting wavelengths generated by the lamp to the fluorochrome on the slide; excitation of the fluorochrome causes it to emit radiation at its emitting wavelengths which are usually longer than the exciting wavelengths.

The objective lens assembly 34 comprises a flat field objective lens 42 followed by a barrier filter 44 adapted to suppress reflected radiation of the exciting wavelengths between the sample and film in the camera while transmitting to the film radiation of the emitting wavelengths. The objective lens assembly 34 provides a magnification at the film well below microscopic levels.

The camera 24 is of the instant development type e.g. Polaroid and comprises a camera back assembly 46, loaded with the film in the plane P.

The removable slide support assembly 32 is adapted to slide between horizontal inverted L-shaped runners 48 fixed in the enclosure 22. The assembly 32 comprises a trapezoidal vertical plate 50 adapted for light-tight engagement with a corresponding aperture in the enclosure 22. Secured to the plate 50 is a pedestal 52 shaped for mating engagement between the runners 48. Mounted on the pedestal 52 is a slide holder 54 in which the slide is held in the operation of the apparatus by a spring-urged arm 56 which retains the slide in position in a slot 59 spaced from the arm 56.

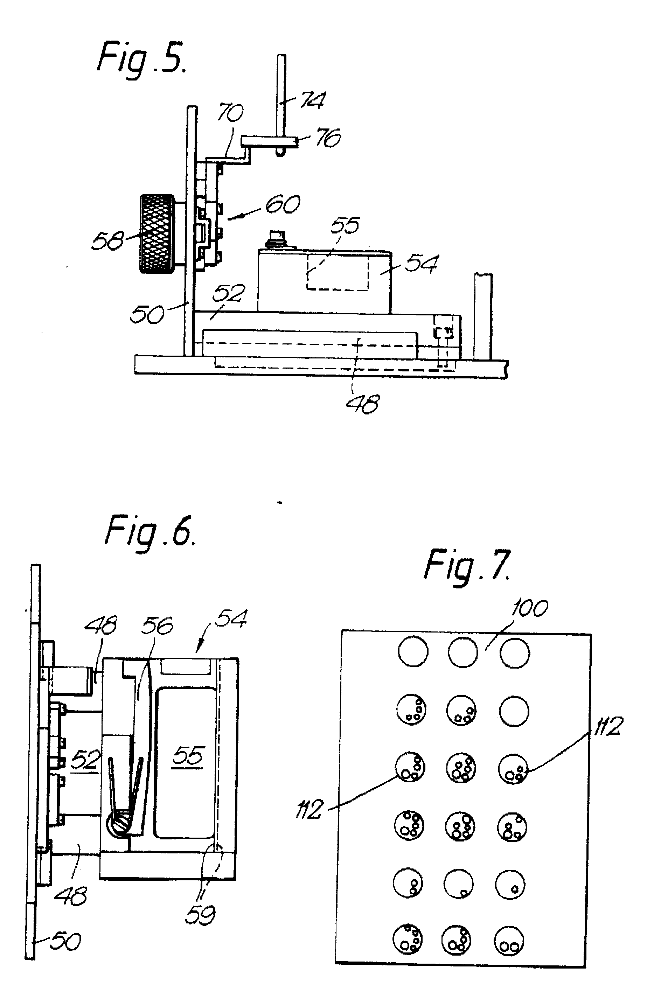

The slide holder 54 comprises a well 55 (Figures 5 and 6) below the plane of the slide; the interior surface of the well 55 is matt, opaque and black and provides an optical background to the slide; a horizontal portion 57 of the interior surface parallel to the slide is spaced therefrom to avoid optical scatter.

The slide support assembly 32 is locked in a closed position in the operation of the apparatus by rotation of a knurled knob 58 mounted on the plate 50. The knob 58 is arranged to operate a locking mechanism comprising a circular disc 60 (Figure 4) mounted on the rear of the plate 50 for rotation by the knob 58. The disc 60 is pivotally linked to sliding bolts 62 which are arranged to slide in brackets 64 between an unlocked position (Figure 4) and a locked position in which the bolts 62 engage behind portions of the enclosure 22 which define the aperture in the enclosure which is closed by the plate 50. It will be realised that anti-clockwise (Figure 4) rotation of the disc 60 moves the bolts 62 from their unlocked position to their locked position.

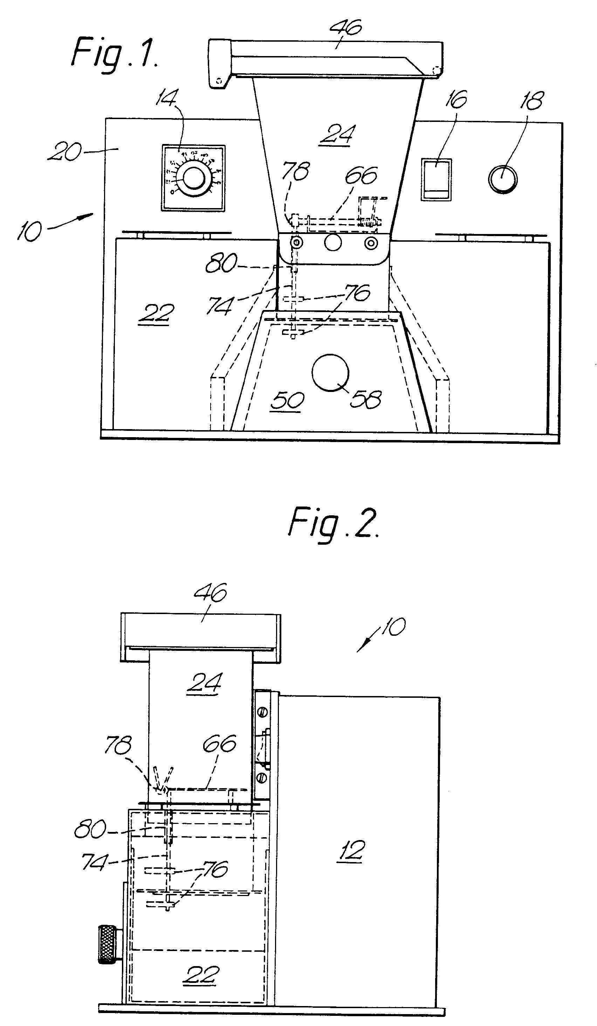

This anti-clockwise rotation of the disc 60 also operates a shutter 66 (Figures 1 and 2) of the camera 24 via a pivotal link 68 (Figures 4 and 5), a lever 70 pivoted at, 72 and a push rod 74, a collar 76 of which engages the lever 70 remotely from the pivot 72. Upper and lower positions of the collar 76 are shown in Figures 1 and 2.

The shutter 66 is hinged at 78 and is spring urged into a horizontal, closed, position. The push rod 74 extends through a guide 80 to engage the shutter to the right (Figure 2) of the hinge 78, and it will be realized that anti-clockwise rotation of the disc 60 by operation of the knob 58 lifts the push rod 74 via the link 68, lever 70 and collar 76 to open the shutter 66 about its hinge 78, so that there is a clear optical path from the slide to the film.

The operation of the apparatus will now be summarised from a condition in which the slide support assembly 32 has been removed from the enclosure 22, the camera shutter 66 is closed, and light cannot enter the camera 24.

A slide containing a selectively fluorescent sample for assay is mounted on the slide holder 54; the support assembly is slid into the enclosure 22 and locked in its closed position by rotation of the knob 58, which simultaneously causes opening of the shutter 66; but since the enclosure 22 is now light-tight, light can still not enter the camera 24.

The power is now switched on by operation of the switch 16, the desired exposure is set on the timer 14, and the push button 18 is pressed to initiate the exposure cycle which commences with switching on the lamps 26, and terminates with them being switched off by operation of the timer 14. The exposed film is developed in the usual manner and removed from the camera back 46. Images of the sample are recorded on the developed film and where they clearly show areas of specially enhanced fluorescence provide for a rapid objective assessment of the assay results.

Alternative arrangements of the apparatus are envisaged whereby, for example, the power supply is provided by internal or external primary or secondary batteries and appropriately rated lamps are selected according to the power supply. Moreover, for processing large numbers of slides the slide support assembly would be replaced by means for sequentially moving one of a series of slides into position beneath the camera, light sealing the exposure chamber and initiating exposure before expelling that slide and drawing another slide into position.

The Table illustrates by way of example approximate exciting and emitting wavelengths from which appropriate exciting and barrier filters may be selected for use with various fluorochromes.

## TABLE

| Fluorochrome | Excitation Range Colour | Excitation | Emission |
|---|---|---|---|
| Fluorescein isothiocyanate | blue | 450-490 nm | >515 nm |
| Tetramethyl rhodamine isothiocynate | green | 515-565 nm | >590 nm |
| Lissamine rhodamine B | green | ca. 546 nm | >590 nm |
| Dinaphthyl aminosulfonic acid | UV | ca. 365 nm | >397 nm |
| Bisaminophenyl oxadiazole | UV | ca. 365 nm | >397 nm |
| Acriflavine | blue-violet | ca. 436 nm | >520 nm |
| Pararosaniline | green | ca. 546 nm | >590 nm |
| Fluoroescamine | UV | ca. 345 nm | >397 nm |
| Mepacrine | blue-violet | 395-440 nm | >470 nm |
| Dansyl chloride | UV | ca. 365 nm | >397 nm |
| Sulphaflavine | UV | ca. 365 nm | > 397 nm |
| Euchrysine | blue-violet | ca. 436 nm | >520 nm |
| Thioflavine S | blue-violet | ca. 436 nm | >520 nm |
| Coriphosphine | blue | 450-490 nm | >520 nm |
| Methyl green pyronine stilbene | green | ca. 546 nm | >590 nm |
| Acridine orange | blue | 450-490 nm | >520 nm |
| Berberine sulphate | blue-violet | ca. 436 nm | >520 nm |
| Mithramycine | UV-violet | 390-420 nm | >450 nm |
| Phosphine 3 R | blue-violet | ca. 436 nm | >520 nm |
| Aurophosphine G | blue-violet | ca. 436 nm | >520 nm |
| Thiazine red R | green | 515-565 nm | >590 nm |
| Auramine | blue | 450-490 nm | >520 nm |
| Acridine yellow | blue blue-violet | 436-490 nm | >520 nm |
| Primuline 0 | blue-violet | 395-440 nm | >470 nm |

TABLE (continued)

| | | | |
|---|---|---|---|
| Tetracycline | blue-violet blue | 395-440 nm | >470 nm |
| Calceine | UV | ca. 365 nm | >397 nm |
| Xylene orange | green | ca. 546 nm | >590 nm |
| Acid fuchsine | green | ca. 546 nm | >590 nm |
| Quinacrine | blue-violet | ca. 436 nm | >470 |

Example

An indirect (sandwich assay was carried out for chlamydial antibodies. A transparent glass slide (Figure 7) of the usual rectangular form was provided with highly clean glass coated with Teflon (100). Eighteen evenly spaced circular wells 112 were provided on the slide, the wells 112 being free of the Teflon coating, which is provided to prevent spreading of liquid outside the wells during the testing procedure.

Each well 112 contains six discrete micro-dots; each of five of the dots comprises a different variety of purified immobilised chlamydial antigen and the sixth is a negative control free of chlamydial antigen.

In carrying out the procedure a human serum to be tested was introduced into a well 112, incubated at 37°C, washed off which phosphate buffered saline pH 7.3, and dried; any antibodies in the serum complementary to the antigens of the dots were retained on the dots by the antigen-antibody reaction; swine anti-human IgG conjugated with fluorescein isothiocyanate was then introduced into the well 112, again incubated at 37°C washed off with phosphate buffered saline pH 7.3, and dried; the conjugated anti-serum attached to any serumm antibodies retained on the dots. Different dilutions of the serum were used in 17 of the respective different wells 112, and a positive control serum known to contain chlamydial antibodies was used in the eighteenth well.

For further details reference may be made for example to Forsey and Darougar, Journal of Clinical Pathology 33 pages 171-176 (1980) referred to hereinabove.

The slide was then photographed in the apparatus described with reference to Figures 1-6 of the drawings with an exposure of 7.5 seconds, aperture f8, magnification 2x and monochromatic film 20,000 ASA to give the results illustrated in Figure 7. The bright dots are images of selectively fluorescent areas of the sample and indicate positive readings.

The exciting and barrier filters used were Zeiss (West Germany) 48 77 09 BP (Pass Band) 450-490 nm and BP (Pass Band) 515-565 nm respectively. It will be realized that the exciting radiation is in the blue range.

Similar results were obtained in detecting antibodies against herpes simplex, adenoviruses, and human immunodeficiency virus (AIDS).

In a modification each well contains seven discrete micro-dots each containing a respective different antigen: thus one dot contains chlamydial antigen; another, adenovirus antigen; another, herpes simplex virus antigen; another, human immunodeficiency virus antigen (AIDS); another, hepatitis B antigen; another, syphilis antigen, and the remaining one, mycoplasma antigen, This provides for a simultaneous assay for the seven corresponding antibodies.

The method may be amended in order simultaneously to test different sera or even to test a dilution series of each of serveral sera.

**Claims**

1. A diagnostic test process comprising:
   (a) contacting a test patch with a fluid sample suspected to contain one or more specific binding agents, the test patch consisting of an area for receiving the fluid sample and a plurality of spots immobilised therein comprising at least one control spot and at least one test spot, the test spot or spots each comprising a specific binding partner for a specific binding agent to be detected,
   (b) permitting any specific binding agents in the sample to react with or adhere to their complementary specific binding partners on the test patch,
   (c) labelling with a fluorescent label any test spot which has reacted with, or to which is adhering, a specific binding agent,

(d) stimulating such labelled test spots to fluoresce,

(e) photographically recording any fluorescence from the test patch such that a single photographic frame contains an image of the fluorescence from the whole of at least one test patch and such that the image is formed at a magnification of 1 to 10 x and is on a scale such that each fluorescent labelled test spot within the test patch is directly readable by an unaided human eye.

2. A process according to claim 1 wherein the magnification is 1.5 to 5 x.

3. A process according to claim 1 or claim 2 wherein the process is conducted using a microscope slide bearing an array of up to 6 x 3 test patches each containing up to 18 spots including at least one control spot.

4. Apparatus for recording results of a fluorescent test according to any one of claims 1 to 3 comprising:

a sample holder;

a source of exciting radiation positioned to illuminate a selectively fluorescent sample when held in the sample holder;

photographic recording means for recording fluorescent radiation emitted by a sample in the holder; and

means for suppressing radiation of exciting wavelengths disposed between the sample holder and the recording means.

5. Apparatus according to claim 4 wherein the photographic recording means is an instant development film and associated focussing lenses.

6. A process for recording the results of an assay according to any one of claims 1 to 3 comprising subjecting a sample selectively stained with a fluorochrome to exciting radiation so that the fluorochrome is excited to emit radiation at its emitting wavelength, photographically recording the emitted radiation on a photographic film and suppressing radiation of exciting wavelengths, on a path between the sample and the film.

7. A process for recording the results of a test according to claim 6 comprising using an apparatus according to claim 4 or claim 5.

**Patentansprüche**

1. Diagnostisches Testverfahren, bei dem man:

(a) einen Testbereich mit einer fluiden Probe in Kontakt bringt, von der angenommen wird, daß sie ein oder mehrere spezifische bindende Agenzien enthält, wobei der Testbereich aus einem Bereich zur Aufnahme der fluiden Probe und aus einer Vielzahl von darin immobilisierten Punkten besteht, die mindesten einen Vergleichspunkt und mindestens einen Testpunkt aufweisen, wobei der oder die Testpunkt(e) jeweils einen spezifischen Bindungspartner für das festzustellende spezifische bindende Agens aufweisen;

(b) beliebige spezifische bindende Agenzien in der Probe mit ihren komplementären spezifischen Bindungspartnern auf dem Testbereich reagieren oder sich daran anhängen läßt;

(c) alle Testpunkte, mit denen ein spezifisches bindendes Agens reagiert oder sich daran angehängt hat, mit einer Fluoreszenzmarkierung markiert;

(d) die markierten Testpunkte zu Fluoreszenz anregt;

(e) alle Fluoreszenz aus dem Testbereich photographisch so aufzeichnet, daß ein einzelnes photographisches Bild ein Fluoreszenzbild aus dem Gesamten mindestens eines Testbereichs enthält und daß das Bild mit einer Vergrößerung von 1 bis 10 x gebildet wird und einen solchen Maßstab aufweist, daß jeder fluoreszenzmarkierte Testpunkt des Testbereichs mit dem unbewaffneten menschlichen Auge unmittelbar lesbar ist.

2. Verfahren nach Anspruch 1, wobei die Vergrößerung 1,5 bis 5 x ist.

3. Verfahren nach Anspruch 1 oder 2, wobei man das Verfahren durchführt unter Verwendung eines Mikroskop-Objektträgers, der eine Reihe von bis zu 6 x 3 Testbereichen trägt, wobei jeder bis zu 18 Punkte enthält und mindestens einen Vergleichspunkt einschließt.

**4.** Vorrichtung zum Aufzeichnen der Ergebnisse eines Fluoreszenztests gemäß einem der Ansprüche 1 bis 3 mit:

einem Probenhalter;

einer Quelle für Anregungsstrahlung, welche so angeordnet ist, daß sie selektiv eine in den Probenhalter gehaltene Fluoreszenzprobe beleuchtet;

einer photographischen Aufzeichnungseinrichtung zum Aufzeichnen der von der Probe im Halter emittierten Fluoreszenzstrahlung; und

einer zwischen dem Probenhalter und der Aufzeichnungseinrichtung angeordneten Einrichtung zum Unterdrücken von Strahlung mit anregenden Wellenlängen.

**5.** Vorrichtung nach Anspruch 4, wobei die photographische Aufzeichnungseinrichtung ein Film mit Sofortbildentwicklung mit zugehörigen Sammellinsen ist.

**6.** Verfahren zum Aufzeichnen der Ergebnisse einer Bestimmung gemäß einem der Ansprüche 1 bis 3, bei dem man eine selektiv mit einem Fluorochrom gefärbte Probe mit anregender Strahlung belichtet, so daß das Fluorochrom zum Emittieren von Strahlung seiner Emissionswellenlänge angeregt wird, die emittierte Strahlung auf einem photographischen Film photographisch aufzeichnet und auf einem Lichtweg zwischen der Probe und dem Film Strahlung mit anregenden Wellenlängen unterdrückt.

**7.** Verfahren zum Aufzeichnen der Ergebnisse eines Tests gemäß Anspruch 6 unter Verwendung einer Vorrichtung gemäß Anspruch 4 oder 5.

**Revendications**

**1.** Procédé de test diagnostique comprenant les étapes consistant à:

(a) mettre en contact une plage de test avec un échantillon fluide susceptible de contenir un ou plusieurs agents de liaison spécifiques, la plage de test comprenant une zone destinée à recevoir l'échantillon fluide et une pluralité de taches immobilisées dans celui-ci, comprenant au moins une tache témoin et au moins une tache de test, la ou les taches de test comprenant chacune un partenaire de liaison spécifique pour un agent de liaison spécifique à détecter,

(b) permettre à tous les agents de liaison spécifiques de l'échantillon de réagir avec, ou adhérer à, leur partenaire de liaison spécifique complémentaire sur la plage de test,

(c) marquer avec un marqueur fluorescent toute tache de test qui a réagi avec, ou à qui adhère un agent de liaison spécifique,

(d) stimuler la fluorescence de ces taches de test ainsi marquées,

(e) réaliser un enregistrement photographique de toute fluorescence provenant de la plage de test, de façon qu'un seul cliché contienne une image de la fluorescence de la totalité d'au moins une plage de test, et de façon que l'image soit formée avec un agrandissement de 1 à 10 x, et à une échelle telle que chaque tache de test marquée à fluorescente, de la plage de test soit lisible directement à l'oeil nu par un observateur humain.

**2.** Procédé selon la revendication 1, dans lequel l'agrandissement est 1,5 à 5 x.

**3.** Procédé selon la revendication 1 ou 2, dans lequel le procédé est réalisé à l'aide d'une lame de microscope sur laquelle sont disposées jusqu'à 6 x 3 plages de test, dont chacune contient jusqu'à 18 taches, y compris au moins une tache témoin.

**4.** Appareil pour l'enregistrement des résultats d'un test par fluorescence, selon l'une quelconque des revendications 1 à 3, comprenant :

un porte-échantillon ;

une source de rayonnement d'excitation placée de façon à illuminer un échantillon à fluorescence sélective lorsqu'il est porté par le porte-échantillon ;

un moyen d'enregistrement photographique pour enregistrer le rayonnement fluorescent émis par un échantillon se trouvant dans le porte-échantillon ; et

un moyen pour supprimer le rayonnement à des longueurs d'onde d'excitation, disposé entre le porte-échantillon et le moyen d'enregistrement.

**5.** Appareil selon la revendication 4, dans lequel le moyen d'enregistrement photographique est constitué d'une pellicule à développement instantané et de lentilles de mise au point associées.

6. Procédé pour enregistrer les résultats d'un dosage selon l'une quelconque des revendication 1 à 3, comprenant les étapes consistant à soumettre un échantillon sélectivement coloré à l'aide d'un agent fluorochrome à un rayonnement d'excitation, de façon que l'agent fluorochrome soit excité pour émettre un rayonnement à sa longueur d'onde d'émission, à réaliser un enregistrement photographique du rayonnement émis sur une pellicule photographique et à supprimer le rayonnement à des longueurs d'onde d'excitation à un position située sur un trajet entre l'échantillon et la pellicule.

7. Procédé pour l'enregistrement des résultats d'un dosage selon la revendication 6, comprenant l'utilisation d'un appareil selon la revendication 4 ou 5.

Fig.1.

Fig.2.

## Fig.3.

## Fig.4.

Fig.5.

Fig.6.

Fig.7.